# EUROPÄISCHE PATENTSCHRIFT

(11) **EP 3 370 819 B1**
(45) Veröffentlichungstag und Bekanntmachung des Hinweises auf die Patenterteilung: **15.01.2020**
(21) Anmeldenummer: 16787874.3
(22) Anmeldetag: 27.10.2016
(51) Int. Cl.: A61M 39/10, F16L 19/00

(54) **ANSCHLUSSSYSTEM MIT ÜBERWURFMUTTER**
ATTACHMENT SYSTEM WITH SLEEVE NUT
SYSTÈME DE RACCORDEMENT POURVU D'ÉCROUS DE RACCORD

(30) Priorität: 03.11.2015 DE 102015118813
(43) Veröffentlichungstag der Anmeldung: 12.09.2018
(73) Patentinhaber: Hopf, Hans-Jürgen, 90513 Zirndorf (DE)
(72) Erfinder: HOPF, Hans-Jürgen, 90513 Zirndorf (DE); HOPF, Michael, 90513 Zirndorf (DE); HOPF, Alexander, 90491 Nürnberg (DE); KASSAI, Norbert, 90522 Oberasbach (DE)
(74) Vertreter: Wittmann, Ernst-Ulrich
(86) Internationale Anmeldenummer: PCT/EP2016/075959
(87) Internationale Veröffentlichungsnummer: WO 2017/076746

(56) Entgegenhaltungen:
- EP-A1- 3 081 253
- WO-A1-94/22520
- WO-A2-02/056958
- DE-A1-102009 019 340
- DE-C1- 19 833 181
- US-A- 5 047 021
- US-A- 5 113 571
- US-A- 6 152 913
- US-A1- 1 923 086
- US-A1- 2015 141 937

## Beschreibung

Die vorliegende Erfindung betrifft ein Anschlusssystem für Fluidverbindungen, wie sie insbesondere in der Medizin und Medizintechnik eingesetzt werden.

Anschlusssysteme für Fluidverbindungen sind im Stand derTechnik bekannt und werden zum Beispiel in der Medizin und Medizintechnik eingesetzt. Hierbei werden die Anschlusssysteme im Infusionsbereich, dem Bereich der künstlichen enteralen Ernährung, bei Spritzen und Injektionsbesteck, bei der Transfusion und für die Zu- bzw. Überleitung von verschiedenen Durchflussmedien und als sogenanntes "Injektions-Equipment" für die medizinische und pharmazeutische Ausrüstung verwendet, welche unter anderem auch aus mehreren Komponenten bestehen, siehe zum Beispiel , Dokumenten US 5.113.571, US 6.152.913 und US 5.047.021.

In der Medizin ist u.a. die absolute Sicherheit in der Bedienung bei einem größtmöglichen Verwechslungsschutz von Spritzen, enteralen Überleitsystemen, Infusionsschläuchen, Kanülen etc. oberstes Gebot. Daher haben sich in der Medizintechnik zunächst der Luer- und derzeit neu der ENfit™-Anschluss gegenüber früher gebräuchlichen Verbindungssystemen durchgesetzt. Der Luer-Anschluss ist ein Verbindungssystem für Kanülen, Spritzen, Kathetern und Infusionsschläuchen im parenteralen medizinischen Bereich, genormtes durch den ISO 594-2 und den DIN EN 1707 Standard. Der ENFit™-Anschluss ist ein Verbindungssystem für Ernährungssonden, Spritzen und Aufziehhalmen, und Überleitsystemen im enteralen medizinischen Bereich, genormtes durch den DIN EN ISO/IEC 80369-3 Standard.

Die Dichtung wird hierbei durch eine kegelförmige Konstruktion der Verbindungsteile (des Anschluss-Konus) erreicht. Dabei wird der Innenkegel der einen Verbindungsseite auch als "weiblich" bezeichnet, der Außenkegel der Gegenseite als "männlich" bezeichnet. Insbesondere weist der Anschluss-Konus eines Luer-Anschlusses eine genormte Steigung von 6 % auf.

Wenn der Anschluss-Konus zur Sicherung bzw. Verriegelung der Verbindung gegen versehentliches Lösen mit einer Gewindemutter bzw. Überwurfmutter mit Gewinde erweitert ist, bezeichnet man gemäß der Erfindung den Luer-Anschluss und den ENfit™-Anschluss als Lock-Anschluss. Die Version des Luer-Anschlusses ohne Schraubgewinde an der Spritzendüse wird als Steck-Anschluss bezeichnet. In einem Lock-Anschluss wird der Innenkegel wenigstens teilweise in die Gewindemutter bzw. in die Überwurfmutter geschraubt.

Der Luer-Anschluss und der ENfit™-Anschluss garantieren die Kompatibilität zwischen verschiedenen Herstellern in dem jeweiligen medizinischen Einsatzgebiet parenteral oder enteral und sind international anerkannt. Weltweit haben sich der Luer-Anschluss und neu der ENfit™-Anschluss für reversible Verbindungen von Spritzen, Kanülen, Infusionsschläuchen und Ernährungssonden, Aufziehhalmen, Überleitsystemen und dergleichen durchgesetzt. Dies stellt einen großen Vorteil, insbesondere im Rahmen internationaler Hilfs- und Katastropheneinsätze dar.

Bekannte Anschlusssysteme, welche den Luer-Anschluss und/oder ENfit™-Anschluss verwenden, weisen den Nachteil auf, dass die Gewindemutter bzw. die Überwurfmutter mit dem Außenkegel einstückig ausgebildet sind. Die Anwesenheit der Gewindemutter bzw. der Überwurfmutter erhöht die Komplexität der Herstellung im Kunststoffspritzguss des Außenkegels, insbesondere dann, wenn die Gewindemutter bzw. Überwurfmutter ein Gewinde aufweist. Außerdem hat diese Bauweise den Nachteil, dass sie die Verwendungsmöglichkeit des Außekegels beschränkt. Tatsächlich kann ein Außenkegel, welcher durch ein erstes Anschlusssystem verwendet wird, nicht durch ein zweites Anschlusssystem verwendet werden, welches den gleichen Außenkegel, aber einer anderen Gewindemutter bzw. Überwurfmutter bedarf. Der mit der Gewindemutter bzw. der Überwurfmutter einstückig ausgebildete Außenkegel für das zweite Anschlusssystem soll somit durch einen anderen Kunststoffspritzguss-Prozess hergestellt werden, was zu einer deutlichen Erhöhung der Herstellungskosten führt.

Luer-Anschluss und/oder ENfit™-Anschluss verwendende Anschlusssysteme, welche die Gewindemutter bzw. die Überwurfmutter nicht mit dem Außenkegel einstückig ausbilden, sind im Stand der Technik bekannt. Jedoch weisen sie den Nachteil auf, dass die Verbindung zwischen dem Außenkegel und der Gewindemutter bzw. der Überwurfmutter drehbeweglich ist. Insbesondere für einen Lock-Anschluss wird die Verschraubung des Innenkegels in der Gewindemutter bzw. in der Überwurfmutter erschwert, da während der Verschraubung die Gewindemutter bzw. die Überwurfmutter sich mit dem Innenkegel drehen kann. Darüber hinaus weist diese Bauweise eine nicht feste und relativ unsichere Verbindung zwischen dem Innenkegel und dem Außenkegel auf, da der Innenkegel in Bezug auf den Außenkegel sich mit der Gewindemutter bzw. der Überwurfmutter drehen kann. Diese Drehung kann das Anschlusssystem beschädigen und somit kann zur Undichtheit führen.

Aufgabe der vorliegenden Erfindung ist es, die im Stand der Technik bekannten Nachteile wenigstens teilweise zu überwinden. Diese Aufgabe wird durch ein Anschlusssystem gemäß Anspruch 1 gelöst. Bevorzugte Ausgestaltungen der Erfindung sind Gegenstand der jeweiligen Unteransprüche.

Demnach betrifft die vorliegende Erfindung ein Anschlusssystem fürfluiddurchströmte Bauteile, welches wenigstens aus einem Verbindungsbauteil und einer Überwurfmutter besteht.

Das Verbindungsbauteil weist wenigstens einen ersten Mantel auf, welcher eine innenliegende rohrförmige Durchführung bildet. Diese Durchführung weist eine vorgegebene Längserstreckung auf, welche sich wenigstens teilweise entlang einer ersten zentralen Achse erstreckt. Der erste Mantel und somit die Durchführung können wenigstens teilweise eine Kegelsymmetrie um die erste Achse aufweisen, d.h. sie können rotationssymmetrisch zur ersten Achse sein. Außerdem weist das Verbindungsbauteil eine Nut auf, welche im ersten Mantel nach außen in Bezug auf die Durchführung angeordnet ist. Die Nut umläuft wenigstens teilweise den ersten Mantel entlang in einem axialen Abschnitt des ersten Mantels. Gemäß der vorliegenden Erfindung ist ein axialer Abschnitt des ersten Mantels ein Abschnitt des ersten Mantels, welcher im Wesentlichen lotrecht zur ersten Achse steht.

Die Überwurfmutter weist wenigstens einen zweiten Mantel auf, welcher einen innenliegenden Aufnahmebereich bildet. Dieser innenliegende Aufnahmebereich weist eine vorgegebene Längserstreckung auf, welche sich wenigstens teilweise entlang einer zweiten zentralen Achse erstreckt. Der zweite Mantel und somit der innenliegende Aufnahmebereich können wenigstens teilweise eine Kegelsymmetrie um die zweite Achse aufweisen, d.h. sie können rotationssymmetrisch zur zweiten Achse sein. Außerdem weist die Überwurfmutter ein Eingriffselement auf, welches am zweiten Mantel nach innen in Bezug auf den innenliegenden Aufnahmebereich angeordnet ist. Das Eingriffselement erstreckt sich wenigstens teilweise entlang eines axialen Abschnitts des zweiten Mantels. Entsprechend der Erfindung ist ein axialer Abschnitt des zweiten Mantels ein Abschnitt des zweiten Mantels, welcher im Wesentlichen lotrecht zur zweiten Achse steht.

Die erste und die zweite Achse sind im Wesentlichen aufeinander angeordnet und die Durchführung des Verbindungsbauteils ist wenigstens teilweise in dem innenliegenden Aufnahmebereich aufgenommen. Außerdem ist das Eingriffselement wenigstens teilweise in der Nut aufgenommen. Darüber hinaus bilden das Eingriffselement und die Nut eine in Bezug auf die erste Achse und/oder in Bezug auf die zweite Achse azimutale Formschlussverbindung zwischen dem Verbindungsbauteil und der Überwurfmutter und eine in Bezug auf die erste Achse und/oder in Bezug auf die zweite Achse axiale Formschlussverbindung zwischen dem Verbindungsbauteil und der Überwurfmutter. Gemäß der vorliegenden Erfindung ist eine in Bezug auf eine gemeinsame Achse azimutale Formschlussverbindung zwischen einem ersten und einem zweiten Element eine Formschlussverbindung, welche eine Rotationsbewegung des ersten Elements in Bezug auf das zweite Element um die gemeinsame Achse verhindert. Außerdem ist entsprechend der Erfindung eine in Bezug auf eine gemeinsame Achse axiale Formschlussverbindung zwischen einem ersten und einem zweiten Element eine Formschlussverbindung, welche eine lineare Verschiebbarkeit des ersten Elements in Bezug auf das zweite Element entlang der gemeinsamen Achse verhindert.

Das Verbindungsbauteil und die Überwurfmutter gemäß der Erfindung können separat und selbständig voneinander hergestellt werden und danach aufgesetzt werden, um das Anschlusssystem fürfluiddurchströmte Bauteile zu bilden. Die Kunststoffspritzguss-Prozesse, welche das Verbindungsbauteil und die Überwurfmutter herstellen, sind somit relativ einfach, insbesondere einfacher als ein Kunststoffspritzguss-Prozess, welcher ein mit der Überwurfmutter einstückig ausgebildetes Verbindungsbauteil herstellt. Darüber hinaus werden die Verwendungsmöglichkeiten sowohl des Verbindungsbauteils, als auch der Überwurfmutter nicht durch diese Bauweise beschränkt.

Die in Bezug auf die erste Achse und/oder in Bezug auf die zweite Achse azimutale und die in Bezug auf die erste Achse und/oder in Bezug auf die zweite Achse axiale Formschlussverbindung zwischen dem Verbindungsbauteil und der Überwurfmutter bilden eine drehfeste Verbindung zwischen dem Verbindungsbauteil und der Überwurfmutter, welche eine leicht durchzuführende, feste und relativ sichere Verbindung des Anschlusssystems gemäß der vorliegenden Erfindung mit einer weiblichen Aufnahme von anderen fluiddurchströmten Bauteilen gewährleistet.

In einer Ausführungsform der Erfindung weist das Anschlusssystem ein Verbindungspaaroder eine Vielzahl von Verbindungspaare von Fügeelemente auf. Diese Verbindungspaare weisen wenigstens ein erstes und ein zweites Fügeelement auf. Das erste bzw. das zweite Fügeelement ist an der Nut bzw. an dem Eingriffselement angeordnet. Das erste Fügeelement ist ein Vorsprung und das zweite Fügeelement ist ein korrespondierendes Aufnahmeelement. Die Rolle der zwei Fügeelemente kann vertauscht sein, d.h. das zweite Fügeelement kann ein Vorsprung und das erste Fügeelement kann ein korrespondierendes Aufnahmeelement sein. Die zwei Fügeelemente können wenigstens teilweise die Verbindung, insbesondere die in Bezug auf die erste Achse und/oder in Bezug auf die zweite Achse azimutale und/oder die in Bezug auf die erste Achse und/oder in Bezug auf die zweite Achse axiale Formschlussverbindung zwischen dem Verbindungsbauteil und der Überwurfmutter verbessern.

Je nach ihrer Anordnung und ihrer Form können die zwei Fügeelemente die azimutale Verbindung zwischen dem Verbindungsbauteil und der Überwurfmutter in Bezug auf die erste Achse und/oder in Bezug auf die zweite Achse ermöglichen. In einer weiteren Ausführungsform der Erfindung können beispielsweise der Vorsprung und das Aufnahmeelement solcher Verbindungspaare so angeordnet sein, dass die Oberflächenform des Vorsprungs wenigstens teilweise der Oberflächenform des vorgenannten Aufnahmeelements entspricht. Außerdem rastet der Vorsprung wenigstens teilweise in das Aufnahmeelement ein und bildet die in Bezug auf die erste Achse und/oder in Bezug auf die zweite Achse azimutale Formschlussverbindung zwischen dem Verbindungsbauteil und der Überwurfmutter.

Vorzugsweise kann die Oberfläche des Vorsprungs einen ersten und einen zweiten Oberflächenbereich aufweisen, welche wenigstens teilweise entlang einer azimutalen Richtung zur ersten und/oder zur zweiten Achse orientiert und wenigstens teilweise zueinander gegenüberliegend ausgerichtet sind. Gemäß der Erfindung ist eine azimutale Richtung zur ersten und/oder zur zweiten Achse ein an der ersten und/oder ein an der zweiten Achse zentrierter orientierter Kreis, welcher in einer Ebene liegt, welche im Wesentlichen lotrecht zur ersten und/oder zur zweiten Achse steht. Der erste bzw. der zweite Oberflächenbereich des Vorsprungs ist an einem dritten bzw. an einem vierten entsprechenden Oberflächenbereich der Oberfläche des Aufnahmeelements angeordnet. Der erste und der zweite Oberflächenbereich bilden somit die in Bezug auf die erste Achse und/oder in Bezug auf die zweite Achse azimutale Formschlussverbindung zwischen dem Verbindungsbauteil und der Überwurfmutter.

Bei einer Ausführungsform der Erfindung ist der Vorsprung am Umfang wenigstens eines Verbindungspaars eine Stufe. Entsprechend der Erfindung ist vorzugsweise eine Stufe ein an der Oberfläche der Nut und/oder an der Oberfläche des Eingriffselements angeordneter Vorsprung, welcher wenigstens eine erste und eine zweite vorgegebene Längserstreckung aufweist. Insbesondere kann einen Bereich der Oberfläche im Wesentlichen eben und entlang einer im Wesentlichen zur ersten und zur zweiten Längserstreckung lotrechten Richtung orientiert sein. Wenigstens ein Teil der Außerkontur des axialen Abschnittes der Stufe ist nach außen in Bezug auf die Durchführung und nach innen in Bezug auf den Aufnahmebereich angeordnet. Dieser Teil ist im Wesentlichen ein Sehnenpolygon und/oder ein Tangentenpolygon und der Umkreismittelpunkt des Sehnenpolygons und/oder der Inkreismittelpunkt des Tangentenpolygons sind im Wesentlichen an der ersten und/oder an der zweiten Achse angeordnet. Gemäß der Erfindung ist ein axialer Abschnitt der Stufe ein Abschnitt der Stufe, welcher im Wesentlichen lotrecht zur ersten und/oder zur zweiten Achse steht. Eine derartige Form des Vorsprungs gewährleistet eine effiziente Verhinderung einer Rotationsbewegung um die erste und/oder um die zweite Achse, da mehrere Ecken des Teils der Außerkontur des axialen Abschnittes der Stufe zur in Bezug auf die erste Achse und/oder in Bezug auf die zweite Achse azimutalen Formschlussverbindung zwischen dem Verbindungsbauteil und der Überwurfmutter beitragen.

In einer weiteren Ausführungsform der Erfindung ist der Vorsprung am Umfang wenigstens eines Verbindungspaars eine Stufe. Wenigstens ein Teil der Außerkontur des axialen Abschnitts der Stufe ist nach außen in Bezug auf die Durchführung und nach innen in Bezug auf den Aufnahmebereich angeordnet. Außerdem ist dieser Teil im Wesentlichen symmetrisch unter wenigstens einer diskreten Rotationsgruppe zur ersten und/oder zur zweiten Achse. Gemäß der Erfindung ist eine diskrete Rotationsgruppe eine Rotation oder eine endliche Anzahl von nicht äquivalenten Rotationen. Wenigstens eine Rotation der diskreten Rotationsgruppe soll nicht trivial sein, d.h. ihr Winkel soll nicht ein ganzzahligen Verhältnis von 360° sein. Gemäß der vorgenannten Definition kann beispielsweise der vorgenannte Teil der Außerkontur des axialen Abschnitts der Stufe eine Ellipse (symmetrisch unterzwei nicht äquivalenten Rotationen mit Winkel von 180° und 360°) oder ein gleichseitiges Dreieck (symmetrisch unter drei nicht äquivalenten Rotationen mit Winkel von 120°, 140° und 360°) oder ein gleichseitiges Viereck (symmetrisch unter vier nicht äquivalenten Rotationen mit Winkel von 90°, 180°, 270° und 360°) oder ein Pentagramm (symmetrisch unterfünf nicht äquivalenten Rotationen mit Winkel von 72°, 144°, 216°, 288° und 360°) oder ein gleichseitiges Sechseck (symmetrisch unter sechs nicht äquivalenten Rotationen mit Winkel von 60°, 120°, 180°, 240°, 300° und 360°) oder ein gleichseitiges Polygon mit N Ecken (symmetrisch unter N nicht äquivalenten Rotationen) sein. Außerdem kann der vorgenannte Teil der Außerkontur des axialen Abschnitts der Stufe ein Zahnrad mit N Zähnen sein, welches symmetrisch unter N nicht äquivalenten Rotationen ist.

Eine derartige Form des Vorsprungs gewährleistet eine effiziente Verhinderung einer Rotationsbewegung um die erste und/oder um die zweite Achse, da mehrere Oberflächenbereiche des Vorsprungs zur in Bezug auf die erste Achse und/oder in Bezug auf die zweite Achse azimutalen Formschlussverbindung zwischen dem Verbindungsbauteil und der Überwurfmutter beitragen. Außerdem erhöht die Anwesenheit der Symmetrie unter einer diskreten Rotationsgruppe die vorgegebenen Verbindungsmöglichkeiten zwischen dem Verbindungsbauteils und der Überwurfmutter und vereinfacht somit den Aufbauprozess des Anschlusssystems gemäß der vorliegenden Erfindung.

Der Vorsprung wenigstens eines Verbindungspaars kann eine Feder sein. Bei einer Ausführungsform der vorliegenden Erfindung kann sich diese Feder im Wesentlichen entlang einer radialen Richtung zur ersten und/oder zur zweiten Achse erstrecken. Entsprechend der Erfindung ist eine radiale Richtung eine Gerade, welche lotrecht zur ersten und/oder zweiten Achse steht. Solch ein Vorsprung ist am einfachsten herzustellen und ist so angeordnet, dass zwei relativ große Flächen seiner Oberfläche entlang einer azimutalen Richtung orientiert und wenigstens teilweise zueinander gegenüberliegend ausgerichtet sind. Die Anordnung des Vorsprungs gewährleistet somit dass, eine Rotationsbewegung um die erste und/oder um die zweite Achse verhindert wird, auch wenn die Verbindung zwischen dem Verbindungsbauteil und der Überwurfmutter eine lineare Verschiebbarkeit entlang einer radialen Richtung nicht verhindert. Außerdem erhöht diese Anordnung die Verwendungsmöglichkeit des Verbindungsbauteils bzw. der Überwurfmutter. Tatsächlich kann eine entlang einer azimutalen Richtung erstreckte Feder eines Verbindungsbauteils bzw. einer Überwurfmutter mit verschiedenen Aufnahmeelementen eine in Bezug auf die erste Achse und/oder in Bezug auf die zweite Achse azimutale Formschlussverbindung zwischen dem Verbindungsbauteil und der Überwurfmutter bilden. Somit kann das vorgenannte Verbindungsbauteil bzw. die vorgenannte Überwurfmutter mit verschiedenen Typen von Überwurfmuttern bzw. Verbindungsbauteilen in verschiedenen Typen von Anschlusssystemen verwendet werden.

In einer weiteren Ausführungsform der vorliegenden Erfindung erstreckt sich die vorgenannte Feder im Wesentlichen entlang einer azimutalen Richtung zur ersten und/oder zur zweiten Achse. Solch ein Vorsprung ist am einfachsten herzustellen und seine Anordnung verbessert somit die in Bezug auf die erste Achse und/oder in Bezug auf die zweite Achse azimutale Formschlussverbindung zwischen dem Verbindungsbauteil und der Überwurfmutter, da eine so angeordnete Feder hoch belastbar in azimutaler Richtung ist.

Die in Bezug auf die erste Achse und/oder in Bezug auf die zweite Achse azimutale Formschussverbindung zwischen dem Verbindungsbauteil und der Überwurfmutter wird durch die Verwendung einer Vielzahl von Verbindungspaaren erhöht. Außerdem können die Vorsprünge der Verbindungspaare so aufgebaut werden, dass die Vorsprünge gleich sind und ihre Anordnung symmetrisch unter einer diskreten Rotationsgruppe zur ersten und/oder zur zweiten Achse ist. Diese diskrete Rotationsgruppe erhöht die Zahl der vorgegebenen Verbindungsmöglichkeiten zwischen dem Verbindungsbauteil und der Überwurfmutter und vereinfacht somit den Aufbauprozess des Anschlusssystems gemäß der vorliegenden Erfindung. Insbesondere umfasst die vorgenannte diskrete Rotationsgruppe N nicht äquivalente Rotationen, weist das Anschlusssystem N vorgegebenen Verbindungsmöglichkeiten zwischen dem Verbindungsbauteil und der Überwurfmutter auf.

Beispielsweise sind bei einer Ausführungsform der Erfindung der Vorsprung eines ersten Verbindungspaars und der Vorsprung eines zweiten Verbindungspaars im Wesentlichen gleich und ihre Anordnung ist im Wesentlichen symmetrisch zu einer Achsenspiegelung an der ersten und/oder der zweiten Achse, d.h. ist ihre Anordnung symmetrisch unter einer diskreten Rotationsgruppe zur ersten und/oder zur zweite Achse, welche zwei nicht äquivalenten Rotationen mit Winkel von 180° und 360° umfasst. Insbesondere sind der Vorsprung des ersten Verbindungspaars und der Vorsprung des zweiten Verbindungspaars so angeordnet, dass sie im Wesentlichen ineinander durch eine Achsenspiegelung an der ersten und/oder der zweiten Achse überführt werden können.

Außerdem sind in einer weiteren Ausführungsform der Erfindung der Vorsprung eines dritten Verbindungspaars, der Vorsprung eines vierten Verbindungspaars, der Vorsprung eines fünften Verbindungspaars und der Vorsprung eines sechsten Verbindungspaars im Wesentlichen gleich. Außerdem ist ihre Anordnung im Wesentlichen rotationssymmetrisch mit einem Winkel von 90°, 180°, 270° und 360° zur ersten und/oder zweiten Achse, d.h. ist ihre Anordnung symmetrisch unter einer diskreten Rotationsgruppe zur ersten und/oder zur zweite Achse, welche vier nicht äquivalente Rotationen mit einem Winkel von 90°, 180°, 270° und 360° umfasst. Insbesondere können der Vorsprung des dritten Verbindungspaars und der Vorsprung des vierten Verbindungspaars im Wesentlichen ineinander durch eine Achsenspiegelung an der ersten und/oder der zweiten Achse überführt werden. Außerdem können der Vorsprung des fünften Verbindungspaars und der Vorsprung des sechsten Verbindungspaars im Wesentlichen ineinander durch eine Achsenspiegelung an der ersten und/oder der zweiten Achse überführt werden. Drüber hinaus können der Vorsprung des dritten Verbindungspaars und der Vorsprung des fünften Verbindungspaars im Wesentlichen ineinanderdurch eine Rotation um 90° zur ersten und/oder zweiten Achse überführt werden.

Entsprechend einer weiteren Ausführungsform der vorliegenden Erfindung ist der erste Mantel wenigstens im Bereich eines Endes der Durchführung im Wesentlichen kegelförmig bzw. kegelstumpfförmig. Insbesondere bildet der erste Mantel wenigstens im Bereich eines Endes der Durchführung einen Kegel bzw. einen Kegelstumpf, welcher wenigstens teilweise eine Kegelsymmetrie um die erste Achse aufweist. Nimmt das vorgenannte Ende der Durchführung die Verbindung des Anschlusssystems gemäß mit einer weiblichen Aufnahme von anderen fluiddurchströmten Bauteilen teil, vereinfacht die oben beschriebene Form des ersten Mantels diese Verbindung. Außerdem bildet der erste Mantel einen Außenkegel, welcher mit einem entsprechenden Innenkegel von anderen fluiddurchströmten Bauteilen einen Luer-Anschluss oder einen ENfit™-Anschluss erreichen kann.

Bei einer Ausführungsform der Erfindung weist die Überwurfmutter ein Gewinde auf. Dieses Gewinde ist am zweiten Mantel nach innen in Bezug auf den Aufnahmebereich angeordnet und erstreckt sich wenigstens teilweise entlang des axialen Abschnittes des zweiten Mantels. Diese Überwurfmutter kann mit einem Innenkegel von anderen fluiddurchströmten Bauteilen verschraubt werden und kann somit einen Lock-Anschluss bilden.

In einer weiteren Ausführungsform der vorliegenden Erfindung weist die Überwurfmutter am zweiten Mantel Durchbrechungen auf. Diese Durchbrechungen erstrecken sich wenigstens teilweise entlang eines axialen Abschnittes des zweiten Mantels und sind hieran anschließend im zweiten Mantel Stege gebildet, aufweichen die Abschnitte des Gewindes angeordnet sind. Gemäß der vorliegenden Erfindung wird dabei als Steg ein Strukturmerkmal des zweiten Mantels verstanden, welches wenigstens an einer Seite an die umlaufende Durchbrechung anschließt. Darüber hinaus können diese Stege auch an zwei Durchbrechungen anschließen, wobei dies nicht zwingend notwendig ist.

Das so hergestellte Gewinde der Überwurfmutter hat damit den Vorteil, dass für dessen Herstellung kein innerhalb des Gewindes angeordnetes Werkzeug bzw. Werkzeugabschnitt vorgesehen werden muss. Durch einfaches lineares Schließen und Öffnen des Spritzgusswerkzeuges inkl. des ggf. notwendigen Schieber wird somit eine Form für ein innenliegendes Gewinde bereitgestellt, mit welchem die innenliegenden Gewindegänge der Überwurfmutter erzeugt werden können. Diese Art der Herstellung hat den Vorteil, dass keine drehbeweglichen oder ausfaltbaren Werkzeugbestandteile zur Formbildung des Innengewindes benutzt werden müssen und sich somit auch die Zykluszeiten im Spritzgussverfahren reduzieren lassen. Ferner dient es zur Gewichtsreduzierung und Materialeinsparung bei der Herstellung des Produktes.

Gemäß einer Ausführungsform der vorliegenden Erfindung bilden die Abschnitte des Gewindes wenigstens Flanken eines Gewindes. Insbesondere sind die Abschnitte des Gewindes auf die Bereiche der Stege begrenzt. Dies kann an allen Stegen entsprechend vorgesehen sein oder auch nur an einigen dieser Stege. Durch diese Anordnung ergibt sich als Vorteil die Vereinfachung des Herstellungsprozesses, insbesondere des Kunststoffspritzguss-Prozesses. Außerdem wird die Elastizität des Gewindes erhöht, welche nicht nur zur einfacheren Befestigung sondern auch zur besseren Abdichtung der Verbindung zwischen der Überwurfmutter und einem anderen fluiddurchströmten Bauteil führt.

Entsprechend einer weiteren Ausführungsform der vorliegenden Erfindung werden wenigstens Teile des Anschlusssystems aus einem Material hergestellt, das aus einer Gruppe ausgewählt ist, welche duro- und thermoplastische Kunststoff und insbesondere Polyphenylensulfid, Polypropylen, Poly-1-buten, Polyvinylchlorid, Polyvinylidenchlorid, Polymethyl-metaacrylat, Polymethylmethacrylat, Polyacrylnitril, Polystyrol, Polysulfon, Polyacetal, Polyvinylalkohol, Polyvinylacetat, Ethylen-Vinylacetat (EVA), Mischungen aus Ethylen-Vinylacetat und Polyethylen, Ethylen-Vinylacetat-Copolymer (EVAC) wie beispielsweise Elvax oder Evatane, lonomere, Fluorkunststoff, Polyethylen, Polyamid, insbesondere ein teilaromatisches Polyamid, Polycarbonat, Polyester, Polyphenylenoxid, Polysulfon, Polyvinylacetal, Polyurethan, und chlorierter Polyether, Zellulosenitrat, Zelluloseacetat, Zelluloseether, Phenol-Harz, Harnstoff-Harz, Thioharnstoff-Harz, Melamin-Harz, Alkylharz, Allylharz, Silicon, Polyimid, Polybenzimidazol, Epoxidharz, Casein-Kunststoff, vernetztes Polyurethan, ungesättigtes Polyesterharz, antimikrobielle oder antiseptische Materialen wie beispielsweise hochporöses Silber, ionenfrei hergestelltes Silber, Silberverbindungen und insbesondere Microsilber, Metallionen freisetzende Verbindungen, Bisphenol-A-freie Materialien wie zum Beispiel Tritan, Terlux, Acrylnitril-Butadien-Styrol, Kombinationen hiervon und dergleichen umfasst.

Die vorliegende Erfindung umfasst neben dem Anschlusssystem auch die Verwendung des Anschlusssystems für die Herstellung entsprechend fluiddurchströmter Bauteile in der Medizin und der Medizintechnik, wie beispielsweise Infusions- oder Transfusionsschläuche, Y-Verbinder, Mehrwegehähne, Mehrfachverteiler, Injektionsequipment wie Nadeln, Zugänge, enterale Systeme insbesondere Überleitsysteme, Ernährungssonden, Spritzen und Aufziehalme, diverse PEG-Katheter und Jejunal-Katheter oder ähnliches und Kombination hiervon.

Nachfolgend werden verschieden Ausführungsformen der Erfindung erläutert, wobei diese nur Beispielshaft der Erfindung wiedergeben und keine Einschränkung der Erfindungsidee in Bezug auf Abwandlungen darstellen. Dabei zeigt:
- Figur 1a bis Figur 1c: eine erste Ausführungsform des Anschlusssystems 100;
- Figur 2a bis Figur 2c: eine zweite Ausführungsform des Anschlusssystems 100;
- Figur3a bis Figur 3c: eine dritte Ausführungsform des Anschlusssystems 100;
- Figur 4a bis Figur 4c: eine vierte Ausführungsform des Anschlusssystems 100;
- Figur5a bis Figur 5c: eine fünfte Ausführungsform des Anschlusssystems 100;
- Figur 6a bis Figur 6c: eine sechste Ausführungsform des Anschlusssystems 100.

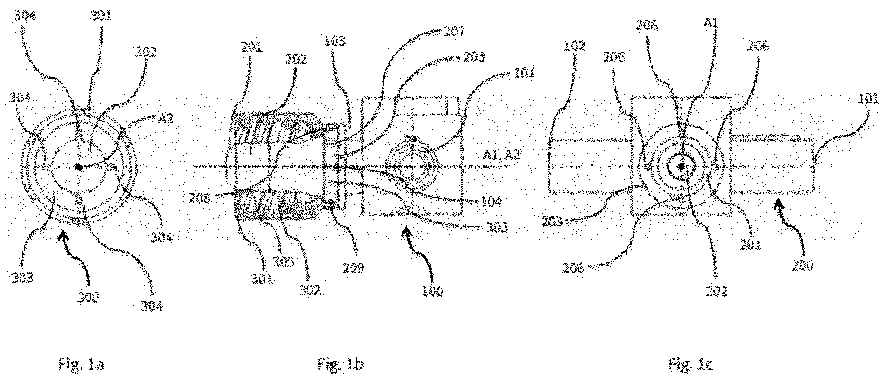

Fig. 1b zeigt einen Längsschnitt der ersten Ausführungsform des Anschlusssystems 100, d.h. einen Abschnitt, welcher in einer Ebene liegt, welche die erste A1 und/oder die zweite A2 Achse umfasst. Fig. 1a bzw. Fig. 1c zeigt einen Querschnitt der Überwurfmutter 300 bzw. des Verbindungsbauteils 200 der ersten Ausführungsform des Anschlusssystems 100. Gemäß der Erfindung ist ein Querschnitt eines Elementes der Anschnitt des Elementes, welcher lotrecht zur ersten Achse A1 und/oder zur zweiten Achse A2 steht.

Das Anschlusssystem 100 bildet einen T-förmigen Drei-Wege-Hahn mit einer ersten Anschlussstelle 101 und einer zweiten Anschlussstelle 102, welche sich gegenüberliegend angeordnet sind, und mit einer dritten Anschlussstelle 103, welche das Verbindungsbauteil 200 und die Überwurfmutter 300 aufweist.

Der erste Mantel 201 des Verbindungsbauteils 200 bildet die innenliegende rohrförmige Durchführung 202. Der erste Mantel 201 und somit die Durchführung 202 weisen eine Kegelsymmetrie um die erste Achse A1 auf. Außerdem ist der erste Mantel 201 im Bereich eines Endes der Durchführung 202 kegelförmig insbesondere kegelstumpfförmig.

Die Nut 203 des Verbindungsbauteils 200 ist im ersten Mantel 201 nach außen in Bezug auf die Durchführung 202 angeordnet und umläuft den ersten Mantel 201 entlang eines axialen Abschnittes des ersten Mantels 201. Die Nut 203 weist eine erste Fläche 207, eine zweite Fläche 208 und eine dritte Fläche 209 auf. Die erste Fläche 207 und die zweite Fläche 208 sind im Wesentlichen eben und im Wesentlichen entlang der ersten Achse A1 orientiert. Anstatt ist die dritte Fläche 209 lotrecht zur ersten Achse A1 orientiert.

Die Nut 203 weist vier im Wesentlichen zueinander gleiche Vorsprünge 206 auf. Diese Vorsprünge 206 sind Federn und erstrecken sich im Wesentlichen parallel zur ersten Achse A1. Die Vorsprünge 206 sind auf der dritten Fläche 209 der Nut 203 angeordnet und sind in Kontakt mit der ersten Fläche 207 und der zweiten Fläche 208 der Nut 203. Der Querschnitt der Vorsprünge 206 ist in Wesentlichen rechteckförmig. Außerdem ist die Anordnung der Vorsprünge 206 im Wesentlichen rotationssymmetrisch mit einem Winkel von 90°, 180°, 270° und 360° zur ersten Achse A1.

Der zweite Mantel 301 der Überwurfmutter 300 bildet den innenliegenden Aufnahmebereich 302. Der zweite Mantel 301 und somit der Aufnahmebereich 302 weisen eine Kegelsymmetrie um die zweite Achse A2 auf, welche im Wesentlichen auf der ersten Achse A1 angeordnet ist. Außerdem ist der zweite Mantel 301 wenigstens teilweise kegelförmig. Die Überwurfmutter weist ein Gewinde 305 auf, welches am zweiten Mantel 301 nach innen in Bezug auf den Aufnahmebereich 302 angeordnet ist. Dieses Gewinde 305 erstreckt sich wenigstens teilweise entlang des axialen Abschnittes des zweiten Mantels 301.

Außerdem weist die Überwurfmutter ein Eingriffselement 303 auf, welches am zweiten Mantel 301 nach innen in Bezug auf den Aufnahmebereich 302 angeordnet ist. Das Eingriffselement 303 erstreckt sich wenigstens teilweise entlang eines axialen Abschnitts des zweiten Mantels 301. Das Eingriffselement 303 weist vier im Wesentlichen zueinander gleiche Aufnahmeelemente 304 auf. Diese Aufnahmeelemente 304 sind Nuten und erstrecken sich im Wesentlichen parallel zurzweiten Achse A2. Die Oberflächenform der Aufnahmeelemente 304 entspricht im Wesentlichen der Oberflächenform derVorsprünge 206 des Verbindungsbauteils 200. Außerdem ist der Querschnitt der Vorsprünge 206 in Wesentlichen rechteckförmig. Darüber hinaus ist die Anordnung der Aufnahmeelemente 304 im Wesentlichen rotationssymmetrisch mit einem Winkel von 90°, 180°, 270° und 360° zur zweiten Achse A2.

Jedes Aufnahmeelement 304 der Überwurfmutter 300 bildet mit einem korrespondierenden Vorsprung des Verbindungsbauteils 200 ein Verbindungspaar 104, welches zur in Bezug auf die erste Achse A1 und/oder in Bezug auf die zweite Achse A2 azimutalen Formschlussverbindung zwischen dem Verbindungsbauteil 200 und der Überwurfmutter 300 beiträgt. Insbesondere rastet jeder Vorsprung 206 wenigstens teilweise in das korrespondierende Aufnahmeelement 304 ein.

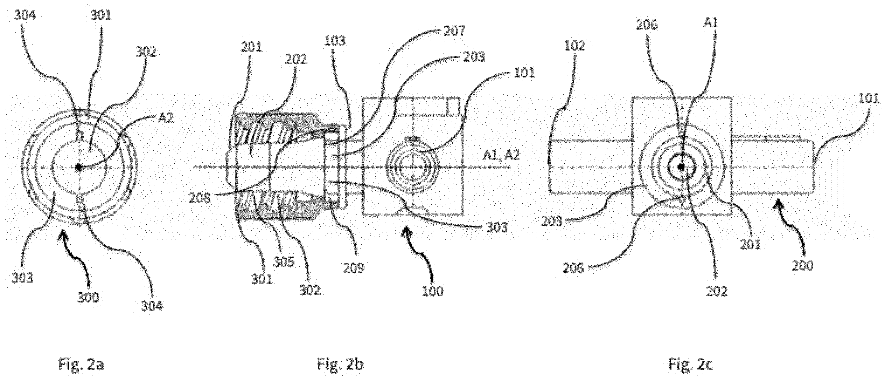

Fig. 2b zeigt einen Längsschnitt der zweiten Ausführungsform des Anschlusssystems 100 und Fig. 2a bzw. Fig. 2c zeigt einen Querschnitt der Überwurfmutter 300 bzw. des Verbindungsbauteils 200 der zweiten Ausführungsform des Anschlusssystems 100. Die zweite Ausführungsform des Anschlusssystems 100 und die erste Ausführungsform des Anschlusssystems 100 unterscheiden sich in der Anzahl ihrer Vorsprünge 206, in der Anzahl ihrer Aufnahmeelemente 304 und in der Symmetrie, welche die Anordnung der Vorsprünge 206 und die Anordnung der Aufnahmeelemente 304 aufweisen.

Tatsächlich weist die Nut 203 der zweiten Ausführungsform des Anschlusssystems 100 zwei im Wesentlichen zueinander gleiche Vorsprünge 206 auf, welche Federn sind und sich im Wesentlichen parallel zur ersten Achse A1 erstrecken. Außerdem weist das Eingriffselement 303 der Überwurfmutter der zweiten Ausführungsform des Anschlusssystems 100 zwei im Wesentlichen zueinander gleiche Aufnahmeelemente 304 auf, welche Nuten sind und sich im Wesentlichen parallel zur zweiten Achse A2 erstrecken.

Die Anordnung der Vorsprünge 206 bzw. der Aufnahmeelemente 304 ist im Wesentlichen symmetrisch zu einer Achsenspiegelung an der ersten Achse A1 bzw. an der zweiten Achse A2. Die Oberflächenform der Aufnahmeelemente 304 entspricht im Wesentlichen der Oberflächenform der Vorsprünge 206 und jedes Aufnahmeelement 304 bildet mit einem korrespondierenden Vorsprung 206 ein Verbindungspaar 104, welches zur in Bezug auf die erste Achse A1 und/oder in Bezug auf die zweite Achse A2 azimutalen Formschlussverbindung zwischen dem Verbindungsbauteil 200 und der Überwurfmutter 300 beiträgt.

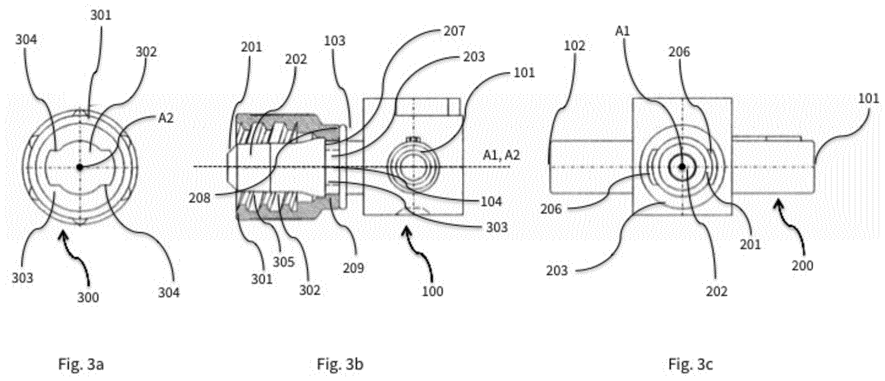

Fig. 3b zeigt einen Längsschnitt der dritten Ausführungsform des Anschlusssystems 100 und Fig. 3a bzw. Fig. 3c zeigt einen Querschnitt der Überwurfmutter 300 bzw. des Verbindungsbauteils 200 der dritten Ausführungsform des Anschlusssystems 100. Die dritte Ausführungsform des Anschlusssystems 100 und die zweite Ausführungsform des Anschlusssystems 100 unterscheiden sich sowohl in der Form des Querschnittes der Vorsprünge 206 als auch in der Form des Querschnittes der Aufnahmeelemente 304. Gemäß der dritten Ausführungsform des Anschlusssystems 100 ist tatsächlich der Querschnitt der zwei Vorsprünge 206 und der zwei Aufnahmeelemente 304 wenigstens teilweise ein Kreissegment.

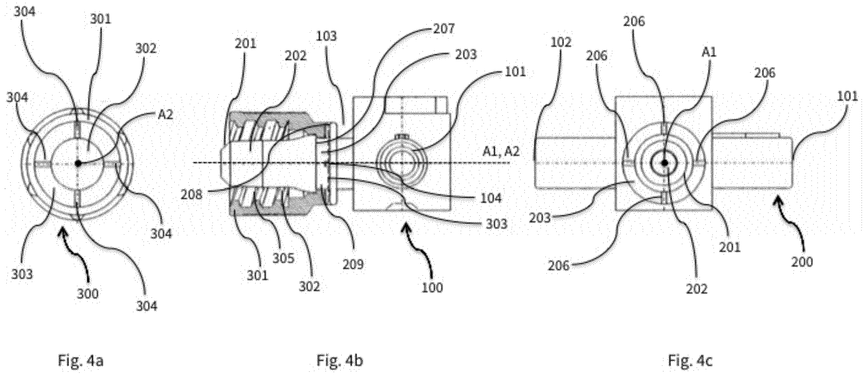

Fig. 4b zeigt einen Längsschnitt der vierten Ausführungsform des Anschlusssystems 100 und Fig. 4a bzw. Fig. 4c zeigt einen Querschnitt der Überwurfmutter 300 bzw. des Verbindungsbauteils 200 der vierten Ausführungsform des Anschlusssystems 100. Die vierte Ausführungsform des Anschlusssystems 100 und die erste Ausführungsform des Anschlusssystems 100 unterscheiden sich sowohl in der Anordnung der Vorsprünge 206 als auch in der Anordnung der Aufnahmeelemente 304.

Die vier zueinander gleichen Vorsprünge 206 der Nut 203 der vierten Ausführungsform des Anschlusssystems 100 sind Federn und erstrecken sich im Wesentlichen entlang einer radialen Richtung zur ersten Achse A1. Die Vorsprünge 206 sind auf der zweiten Fläche 208 der Nut 203 angeordnet und sind in Kontakt mit der dritten Fläche 209 der Nut 203. Außerdem stimmt die radiale Ausdehnung der Vorsprünge 206 mit der radiale Ausdehnung derzweiten Fläche 208 überein.

Die vierzueinander gleichen Aufnahmeelemente 304 des Eingriffselements 303 der vierten Ausführungsform des Anschlusssystems 100 sind Nuten und erstrecken sich im Wesentlichen entlang einer radialen Richtung zur zweiten Achse A2.

Die Anordnung der Vorsprünge 206 bzw. der Aufnahmeelemente 304 ist im Wesentlichen rotationssymmetrisch mit einem Winkel von 90°, 180°, 270° und 360° zur ersten Achse A1 bzw. zur zweiten Achse A2. Die Oberflächenform der Aufnahmeelemente 304 entspricht im Wesentlichen der Oberflächenform der Vorsprünge 206 und jedes Aufnahmeelement 304 bildet mit einem korrespondierenden Vorsprung 206 ein Verbindungspaar 104, welches zur in Bezug auf die erste Achse A1 und/oder in Bezug auf die zweite Achse A2 azimutalen Formschlussverbindung zwischen dem Verbindungsbauteil 200 und der Überwurfmutter 300 beiträgt.

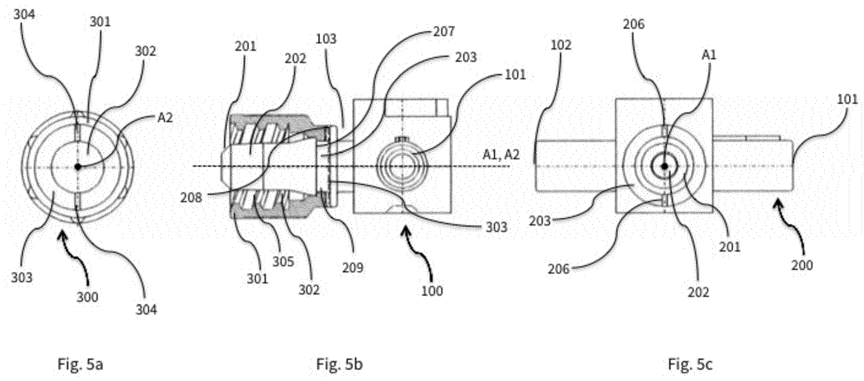

Fig. 5b zeigt einen Längsschnitt derfünften Ausführungsform des Anschlusssystems 100 und Fig. 5a bzw. Fig. 5c zeigt einen Querschnitt der Überwurfmutter 300 bzw. des Verbindungsbauteils 200 derfünften Ausführungsform des Anschlusssystems 100. Die fünfte Ausführungsform des Anschlusssystems 100 und die zweite Ausführungsform des Anschlusssystems 100 unterscheiden sich sowohl in der Anordnung der Vorsprünge 206 als auch in der Anordnung der Aufnahmeelemente 304.

Die zwei zueinander gleiche Vorsprünge 206 der Nut 203 der fünften Ausführungsform des Anschlusssystems 100 sind Federn und erstrecken sich im Wesentlichen entlang einer radialen Richtung zur ersten Achse A1. Die Vorsprünge 206 sind auf der zweiten Fläche 208 der Nut 203 angeordnet und sind in Kontakt mit der dritten Fläche 209 der Nut 203. Darüber hinaus stimmt die radiale Ausdehnung der Vorsprünge 206 mit der radialen Ausdehnung derzweiten Fläche 208 überein.

Die zwei zueinander gleichen Aufnahmeelemente 304 des Eingriffselements 303 der fünften Ausführungsform des Anschlusssystems 100 sind Nuten und erstrecken sich im Wesentlichen entlang einer radialen Richtung zur zweiten Achse A2.

Die Anordnung der Vorsprünge 206 bzw. der Aufnahmeelemente 304 ist im Wesentlichen symmetrisch zu einer Achsenspiegelung an der ersten Achse A1 bzw. an der zweiten Achse A2. Die Oberflächenform der Aufnahmeelemente 304 entspricht im Wesentlichen der Oberflächenform der Vorsprünge 206 und jedes Aufnahmeelement 304 bildet mit einem korrespondierenden Vorsprung 206 ein Verbindungspaar 104, welches zur in Bezug auf die erste Achse A1 und/oder in Bezug auf die zweite Achse A2 azimutalen Formschlussverbindung zwischen dem Verbindungsbauteil 200 und der Überwurfmutter 300 beiträgt.

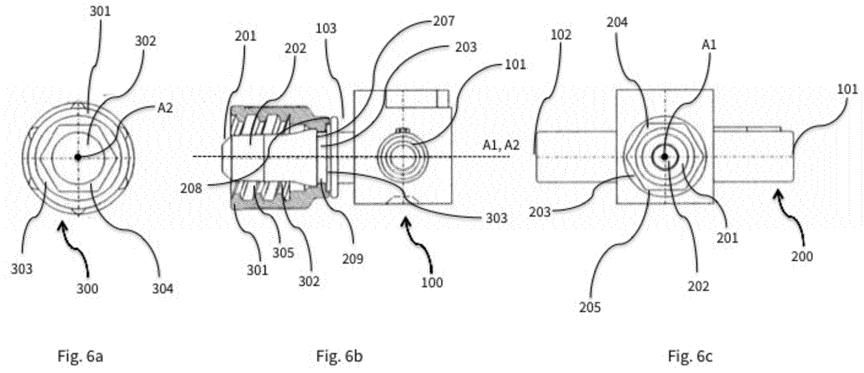

Fig. 6b zeigt einen Längsschnitt der sechsten Ausführungsform des Anschlusssystems 100 und Fig. 6a bzw. Fig. 6c zeigt einen Querschnitt der Überwurfmutter 300 bzw. des Verbindungsbauteils 200 der sechsten Ausführungsform des Anschlusssystems 100. Die sechste Ausführungsform des Anschlusssystems 100 und die erste Ausführungsform des Anschlusssystems 100 unterscheiden sich sowohl in der Anzahl, in der Form und in der Anordnung der Vorsprünge 206 als auch in der Anzahl, in der Form und in der Anordnung der Aufnahmeelemente 304.

Die Nut 203 der sechsten Ausführungsform des Anschlusssystems 100 weist einen Vorsprung 206 auf. Der Vorsprung 206 ist eine Stufe 204, welche auf der zweiten Fläche 208 der Nut 203 angeordnet und in Kontakt mit der dritten Fläche 209 der Nut 203 ist. Der Außerkontur des axialen Abschnitts der Stufe 204 umfasst eine erste Kontur 205 und eine zweite Kontur (nicht dargestellt). Die erste Kontur 205 ist nach außen in Bezug auf die Durchführung 202 und nach innen in Bezug auf den Aufnahmebereich 302 angeordnet. Außerdem ist die erste Kontur 205 im Wesentlichen ein gleichseitiges Sechseck, dessen Umkreismittelpunkt im Wesentlichen an der ersten Achse A1 angeordnet ist. Die erste Kontur 205 ist somit im Wesentlichen symmetrisch unter einer diskreten Rotationsgruppe zur ersten Achse A1. Diese Rotationsgruppe umfasst sechs nicht äquivalente Rotationen mit einem Winkel von 60°, 120°, 180°, 240°, 300° und 360°.

Das Eingriffselement 303 der Überwurfmutter 300 der sechsten Ausführungsform des Anschlusssystems 100 weist ein Aufnahmeelement 304 auf. Die Oberflächenform des Aufnahmeelements 304 entspricht im Wesentlichen der Oberflächenform der Stufe 204 und somit ist das Aufnahmeelement 304 im Wesentlichen symmetrisch unter einer diskreten Rotationsgruppe zur zweiten Achse A2, welche sechs nicht äquivalenten Rotationen mit einem Winkel von 60°, 120°, 180°, 240°, 300° und 360° umfasst. Das Aufnahmeelement 304 bildet mit der Stufe 204 ein Verbindungspaar 104, welches die in Bezug auf die erste Achse A1 und/oder in Bezug auf die zweite Achse A2 azimutale Formschlussverbindung zwischen dem Verbindungsbauteil 200 und der Überwurfmutter 300 bildet.

### Bezugszeichenliste

- 100: Anschlusssystem;
- 101: Erste Anschlussstelle;
- 102: Zweite Anschlussstelle;
- 103: Dritte Anschlussstelle;
- 104: Verbindungspaar;
- 200: Verbindungsbauteil;
- 201: Erster Mantel;
- 202: Durchführung;
- 203: Nut;
- 204: Stufe;
- 205: Erste Kontur der Stufe;
- 206: Vorsprung;
- 207: Erste Fläche der Nut;
- 208: Zweite Fläche der Nut;
- 209: Dritte Fläche der Nut;
- 300: Überwurfmutter;
- 301: Zweiten Mantel;
- 302: Aufnahmebereich;
- 303: Eingriffselement;
- 304: Aufnahmeelement;
- 305: Gewinde.

## Patentansprüche

1. Anschlusssystem für fluiddurchströmte Bauteile (100) bestehend wenigstens aus einem Verbindungsbauteil (200) und einer Überwurfmutter (300), wobei das Verbindungsbauteil (200) wenigstens
• einen ersten Mantel (201) aufweist, welcher eine innenliegende rohrförmige Durchführung (202) mit einer vorgegebenen Längserstreckung bildet, welche sich wenigstens teilweise entlang einer ersten zentralen Achse (A1) erstreckt, und
• eine im ersten Mantel (201) nach außen in Bezug auf die Durchführung (202) angeordnete Nut (203) aufweist, welche wenigstens teilweise den ersten Mantel (201) entlang in einem axialen Abschnitt des ersten Mantels (201) umläuft,
wobei die Überwurfmutter (300) wenigstens
• einen zweiten Mantel (301) aufweist, welcher einen innenliegenden Aufnahmebereich (302) mit einer vorgegebenen Längserstreckung bildet, welche sich wenigstens teilweise entlang einer zweiten zentralen Achse (A2) erstreckt, und
• eine am zweiten Mantel (301) nach innen in Bezug auf den Aufnahmebereich (302) angeordnetes Eingriffselement (303) aufweist, welches sich wenigstens teilweise entlang einem axialen Abschnitt des zweiten Mantels (301) erstreckt,
wobei
• die erste (A1) und die zweite (A2) Achse im Wesentlichen aufeinander angeordnet sind und eine gemeinsame Achse (A1, A2) bilden,
• die Durchführung (202) wenigstens teilweise in dem Aufnahmebereich (302) der Überwurfmutter (300) aufgenommen ist, und
• das Eingriffselement (303) wenigstens teilweise in der Nut (203) aufgenommen ist,
wobei das Eingriffselement (303) und die Nut (203) eine in Bezug auf die gemeinsame Achse (A1, A2) azimutale Formschlussverbindung zwischen dem Verbindungsbauteil (200) und der Überwurfmutter (300) bilden, um eine Rotationsbewegung des Verbindungsbauteils (200) in Bezug auf die Überwurfmutter (300) um die gemeinsame Achse (A1, A2) zu verhindern,
wobei das Anschlusssystem (100) ein Verbindungspaar (104) oder eine Vielzahl von Verbindungspaaren (104) von Fügeelementen aufweist und
die Verbindungspaare (104) wenigstens ein erstes an der Nut (203) angeordnetes Fügeelement und ein zweites am Eingriffselement (303) angeordnetes Fügeelement aufweist, wobei das erste Fügeelement ein Vorsprung (206) bzw. ein Aufnahmeelement (304) ist und das zweite Fügeelement ein korrespondierendes Aufnahmeelement (304) bzw. ein korrespondierender Vorsprung (206) ist, und
wobei der Vorsprung (206) und das Aufnahmeelement (304) der Verbindungspaare (104) so angeordnet sind, dass die Oberflächenform des Vorsprungs (206) wenigstens teilweise der Oberflächenform des Aufnahmeelements (304) entspricht, der Vorsprung (206) wenigstens teilweise in das Aufnahmeelement (304) einrastet und dadurch die in Bezug auf die gemeinsame Achse (A1, A2) azimutale Formschlussverbindung zwischen dem Verbindungsbauteil (200) und der Überwurfmutter (300) bildet, und
eine in Bezug auf die gemeinsame Achse (A1, A2) axiale Formschlussverbindung zwischen dem Verbindungsbauteil (200) und der Überwurfmutter (300) bilden, um eine lineare Verschiebbarkeit des Verbindungsbauteils (200) in Bezug auf die Überwurfmutter (300) zu verhindern,
die Überwurfmutter (300) ein am zweiten Mantel (301) nach innen in Bezug auf den Aufnahmebereich (302) angeordnetes Gewinde (305) aufweist, welches sich wenigstens teilweise entlang des axialen Abschnittes des zweiten Mantels (301) erstreckt,
**dadurch gekennzeichnet, dass**
die Überwurfmutter (300) am zweiten Mantel (301) Durchbrechungen aufweist, welche sich wenigstens teilweise entlang eines axialen Abschnittes des zweiten Mantels (301) erstrecken und hieran anschließend im zweiten Mantel (301) Stege gebildet sind, auf welchen die Abschnitte des Gewindes (305) angeordnet sind.

2. Anschlusssystem (100) nach Anspruch 1, **dadurch gekennzeichnet, dass**
der erste Mantel (201) im Bereich eines Endes der Durchführung (202) im Wesentlichen kegelförmig ist.

3. Anschlusssystem (100) nach Anspruch 1 oder 2, **dadurch gekennzeichnet, dass**
die Abschnitte des Gewindes (305) wenigstens Flanken eines Gewindes (305) bilden und insbesondere die Abschnitte des Gewindes (305) auf die Bereiche der Stege begrenzt sind.

4. Anschlusssystem (100) nach Anspruch 1, wobei der Vorsprung (206) am Umfang wenigstens eines Verbindungspaars (104) eine Stufe (204) ist, **dadurch gekennzeichnet, dass**
wenigstens ein Teil der Außerkontur des axialen Abschnittes (206) der Stufe (204) nach außen in Bezug auf die Durchführung (202) und nach innen in Bezug auf den Aufnahmebereich (302) angeordnet ist, im Wesentlichen ein Sehnenpolygon und/oder ein Tangentenpolygon ist und der Umkreismittelpunkt des Sehnenpolygons und/oder der Inkreismittelpunkt des Tangentenpolygons im Wesentlichen an der ersten Achse (A1) und/oder der zweiten Achse (A2) angeordnet sind.

5. Anschlusssystem (100) nach Anspruch 1 oder 4, wobei der Vorsprung (206) am Umfang wenigstens eines Verbindungspaars (104) einer Stufe (204) ist, **dadurch gekennzeichnet, dass**
wenigstens ein Teil der Außerkontur des axialen Abschnitts (206) der Stufe (204) nach außen in Bezug auf die Durchführung (202) und nach innen in Bezug auf den Aufnahmebereich (302) angeordnet ist, im Wesentlichen symmetrisch unter wenigstens einer diskreten Rotationsgruppe zur ersten (A1) und/oder zur zweite (A2) Achse ist.

6. Anschlusssystem (100) nach einem der Ansprüche 1, 4oder 5, **dadurch gekennzeichnet, dass**
der Vorsprung (206) wenigstens eines Verbindungspaars (104) eine Feder ist, welche sich im Wesentlichen entlang einer radialen Richtung zur ersten (A1) und/oder zweiten (A2) Achse erstreckt.

7. Anschlusssystem (100) nach einem der Ansprüche 1, oder 4 bis 6, **dadurch gekennzeichnet, dass**
der Vorsprung (206) wenigstens eines Verbindungspaars (104) eine Feder ist, welche sich im Wesentlichen entlang einer azimutalen Richtung zur ersten (A1) und/oder zweiten (A2) Achse erstreckt.

8. Anschlusssystem (100) nach einem der Ansprüche 1, oder 4 bis 7, **dadurch gekennzeichnet, dass**
der Vorsprung (206) eines ersten Verbindungspaars (104) und der Vorsprung (206) eines zweiten Verbindungspaars (104) im Wesentlichen gleich sind und ihre Anordnung im Wesentlichen symmetrisch zu einer Achsenspiegelung an der ersten (A1) und/oder der zweiten (A2) Achse ist.

9. Anschlusssystem (100) nach einem der Ansprüche 1, oder 4 bis 8, **dadurch gekennzeichnet, dass**
der Vorsprung (206) eines dritten Verbindungspaars (104), der Vorsprung (206) eines vierten, der Vorsprung (206) eines fünften Verbindungspaars (104) und der Vorsprung (206) eines sechsten Verbindungspaars (104) im Wesentlichen gleich sind und ihre Anordnung im Wesentlichen rotationssymmetrisch mit einem Winkel von 90º, 180º, 270º und 360º zur ersten (A1) und/oder zweiten (A2) Achse ist.

10. Anschlusssystem (100) nach einem der vorstehenden Ansprüche, **dadurch gekennzeichnet, dass**
wenigstens Teile des Anschlusssystems (100) aus einem Material hergestellt sind, das aus einer Gruppe ausgewählt ist, welche duro- und thermoplastische Kunststoff und insbesondere Polyphenylensulfid, Polypropylen, Poly-l-buten, Polyvinylchlorid, Polyvinylidenchlorid, Polymethyl-metaacrylat, Polymethylmethacrylat, Polyacrylnitril, Polystyrol, Polysulfon, Polyacetal, Polyvinylalkohol, Polyvinylacetat, Ethylen-Vinylacetat (EVA), Mischungen aus Ethylen-Vinylacetat und Polyethylen, Ethylen-Vinylacetat-Copolymer (EVAC) wie beispielsweise Elvax oder Evatane, Ionomere, Fluorkunststoff, Polyethylen, Polyamid, insbesondere ein teilaromatisches Polyamid, Polycarbonat, Polyester, Polyphenylenoxid, Polysulfon, Polyvinylacetal, Polyurethan, und chlorierter Polyether, Zellulosenitrat, Zelluloseacetat, Zelluloseether, Phenol-Harz, Harnstoff-Harz, Thioharnstoff-Harz, Melamin-Harz, Alkylharz, Allylharz, Silicon, Polyimid, Polybenzimidazol, Epoxidharz, Casein-Kunststoff, vernetztes Polyurethan, ungesättigtes Polyesterharz, antimikrobielle oder antiseptische Materialen wie beispielsweise hochporöses Silber, ionenfrei hergestelltes Silber, Silberverbindungen und insbesondere Microsilber, Metallionen freisetzende Verbindungen, Bisphenol-A-freie Materialien wie zum Beispiel Tritan, Terlux, Acrylnitril-Butadien-Styrol, Kombinationen hiervon und dergleichen umfasst.

## Claims

1. Connection system for components (100) through which fluid flows, comprising at least one connection component (200) and a union nut (300),
wherein the connection component (200) has at least
• a first cover (201) which forms an internal tubular passage (202) having a predetermined longitudinal extent which extends at least partially along a first central axis (A1), and
• a groove (203) which is arranged in the first cover (201) outwardly with respect to the passage (202) and which extends at least partially around the first cover (201) in an axial portion of the first cover (201),
wherein the union nut (300) has at least
• a second cover (301) which forms an internal receiving region (302) having a predetermined longitudinal extent which extends at least partially along a second central axis (A2), and
• an engagement element (303) which is arranged on the second cover (301) inwardly with respect to the receiving region (302) and which extends at least partially along an axial portion of the second cover (301),
wherein
• the first axis (A1) and the second axis (A2) are arranged substantially one on the other and form a common axis (A1, A2),
• the passage (202) is received at least partially in the receiving region (302) of the union nut (300), and
• the engagement element (303) is at least partially received in the groove (203),
wherein the engagement element (303) and the groove (203) form a positive-locking connection which is azimuthal with respect to the common axis (A1, A2) between the connection component (200) and the union nut (300) in order to prevent a rotation movement of the connection component (200) with respect to the union nut (300) about the common axis (A1, A2), wherein the connection system (100) has a connection pair (104) or a large number of connection pairs (104) of joining elements, and
the connection pairs (104) have at least one joining element which is arranged on the groove (203) and a second joining element which is arranged on the engagement element (303), wherein the first joining element is a projection (206) or a receiving element (304) and the second joining element is a corresponding receiving element (304) or a corresponding projection (206), and
wherein the projection (206) and the receiving element (304) of the connection pairs (104) are arranged in such a manner that the surface shape of the projection (206) at least partially corresponds to the surface shape of the receiving element (304), the projection (206) engages at least partially in the receiving element (304) and thereby forms the positive-locking connection which is azimuthal with respect to the common axis (A1, A2) between the connection component (200) and the union nut (300), and
form a positive-locking connection which is axial with respect to the common axis (A1, A2) between the connection component (200) and the union nut (300) in order to prevent a linear displaceability of the connection component (200) with respect to the union nut (300),
the union nut (300) has a thread (305) which is arranged on the second cover (301) inwardly with respect to the receiving region (302) and which extends at least partially along the axial portion of the second cover (301),
**characterised in that**
the union nut (300) has on the second cover (301) apertures which extend at least partially along an axial portion of the second cover (301) and adjacent thereto there are formed in the second cover (301) webs on which the portions of the thread (305) are arranged.

2. Connection system (100) according to claim 1,
**characterised in that** the first cover (201) is substantially conical in the region of one end of the aperture (202).

3. Connection system (100) according to claim 1 or claim 2,
**characterised in that** the portions of the thread (305) form at least flanks of a thread (305) and in particular the portions of the thread (305) are limited to the regions of the webs.

4. Connection system (100) according to claim 1, wherein the projection (206) on the periphery of at least one connection pair (104) is a step (204), **characterised in that**
at least a portion of the outer contour of the axial portion (206) of the step (204) is arranged outwardly with respect to the passage (202) and inwardly with respect to the receiving region (302), is substantially a chord polygon and/or a tangent polygon and the circumcentre of the chord polygon and/or the incentre of the tangent polygon are arranged substantially on the first axis (A1) and/or the second axis (A2) .

5. Connection system (100) according to claim 1 or claim 4, wherein the projection (206) on the periphery of at least one connection pair (104) of a step (204), **characterised in that** at least a portion of the outer contour of the axial portion (206) of the step (204) is arranged outwardly with respect to the passage (202) and inwardly with respect to the receiving region (302), is substantially symmetrical under at least one discrete rotational group with respect to the first axis (A1) and/or second axis (A2).

6. Connection system (100) according to any one of claims 1, 4 or 5, **characterised in that**
the projection (206) of at least one connection pair (104) is a spring which extends substantially in a radial direction with respect to the first axis (A1) and/or second axis (A2).

7. Connection system (100) according to any one of claims 1 or 4 to 6, **characterised in that**
the projection (206) of at least one connection pair (104) is a spring which extends substantially in an azimuthal direction with respect to the first axis (A1) and/or second axis (A2).

8. Connection system (100) according to any one of claims 1 or 4 to 7, **characterised in that**
the projection (206) of a first connection pair (104) and the projection (206) of a second connection pair (104) are substantially identical and their arrangement is substantially symmetrical relative to an axial reflection on the first axis (A1) and/or the second axis (A2).

9. Connection system (100) according to any one of claims 1 or 4 to 8, **characterised in that**
the projection (206) of a third connection pair (104), the projection (206) of a fourth connection pair, the projection (206) of a fifth connection pair (104) and the projection (206) of a sixth connection pair (104) are substantially identical and their arrangement is substantially rotationally symmetrical at an angle of 90°, 180°, 270° and 360° with respect to the first axis (A1) and/or second axis (A2).

10. Connection system (100) according to any one of the preceding claims, **characterised in that**
at least portions of the connection system (100) are produced from a material which is selected from a group which comprises thermosetting and thermoplastic plastics material and in particular polyphenylene sulphide, polypropylene, poly-1-butene, polyvinyl chloride, polyvinylidene chloride, polymethyl metaacrylate, polymethyl methacrylate, polyacrylonitrile, polystyrene, polysulphone, polyacetal, polyvinyl alcohol, polyvinyl acetate, ethylene vinyl acetate (EVA), admixtures of ethylene vinyl acetate and polyethylene, ethylene vinyl acetate copolymer (EVAC), such as, for example, Elvax or Evatane, ionomers, fluoroplastics material, polyethylene, polyamide, in particular a partially aromatic polyamide, polycarbonate, polyester, polyphenylene oxide, polysulphone, polyvinyl acetal, polyurethane and chlorinated polyether, cellulose nitrate, cellulose acetate, cellulose ether, phenol resin, urea resin, thiourea resin, melamine resin, alkyl resin, allyl resin, silicone, polyimide, polybenzimidazole, epoxy resin, caseine plastics material, cross-linked polyurethane, unsaturated polyester resin, antimicrobial or antiseptic materials, such as, for example, highly porous silver, silver produced in an ion-free manner, silver compounds and in particular microsilver, compounds which release metal ions, bisphenol-A-free materials, such as, for example, Tritan, Terlux, acrylonitrile-butadiene-styrene, combinations thereof and the like.

## Revendications

1. Système de raccordement pour des composants traversés par un fluide (100) constitué au moins d'un composant de liaison (200) et d'un écrou-raccord (300),
dans lequel le composant de liaison (200) présente au moins
- une première gaine (201) qui forme un passage (202) tubulaire intérieur avec une étendue longitudinale prédéfinie, qui s'étend au moins partiellement le long d'un premier axe central (A1), et
- une rainure (203) agencée dans la première gaine (201) vers l'extérieur par rapport au passage (202), qui fait le tour au moins partiellement de la première gaine (201) le long d'une section axiale de la première gaine (201),
dans lequel l'écrou-raccord (300) présente au moins
- une seconde gaine (301) qui forme une zone de réception (302) intérieure avec une étendue longitudinale prédéfinie, qui s'étend au moins partiellement le long d'un second axe central (A2), et
- un élément de mise en prise (303) agencé au niveau de la seconde gaine (301) vers l'intérieur par rapport à la zone de réception (302), qui s'étend au moins partiellement le long d'une section axiale de la seconde gaine (301),
dans lequel
- le premier (A1) et le second (A2) axes sont agencés sensiblement l'un sur l'autre et forment un axe commun (A1, A2),
- le passage (202) est reçu au moins partiellement dans la zone de réception (302) de l'écrou-raccord (300), et
- l'élément de mise en prise (303) est reçu au moins partiellement dans la rainure (203),
dans lequel l'élément de mise en prise (303) et la rainure (203) forment une liaison par complémentarité de forme azimutale par rapport à l'axe commun (A1, A2) entre le composant de liaison (200) et l'écrou-raccord (300) afin d'empêcher un mouvement de rotation du composant de liaison (200) par rapport à l'écrou-raccord (300) autour de l'axe commun (A1, A2),
dans lequel le système de raccordement (100) présente une paire de liaison (104) ou une pluralité de paires de liaison (104) d'éléments d'assemblage et
les paires de liaison (104) présentent au moins un premier élément d'assemblage agencé au niveau de la rainure (203) et un second élément d'assemblage agencé au niveau de l'élément de mise en prise (303), dans lequel le premier élément d'assemblage est une saillie (206) ou un élément de réception (304) et le second élément d'assemblage est un élément de réception (304) correspondant ou une saillie (206) correspondante, et
dans lequel la saillie (206) et l'élément de réception (304) des paires de liaison (104) sont agencés de sorte que la forme de surface de la saillie (206) corresponde au moins partiellement à la forme de surface de l'élément de réception (304), la saillie (206) s'encliquète au moins partiellement dans l'élément de réception (304) et ainsi forme la liaison par complémentarité de forme azimutale par rapport à l'axe commun (A1, A2) entre le composant de liaison (200) et l'écrou-raccord (300), et
forment une liaison par complémentarité de forme axiale par rapport à l'axe commun (A1, A2) entre le composant de liaison (200) et l'écrou-raccord (300) afin d'empêcher une mobilité linéaire du composant de liaison (200) par rapport à l'écrou-raccord (300),
l'écrou-raccord (300) présente un filet (305) agencé au niveau de la seconde gaine (301) vers l'intérieur par rapport à la zone de réception (302), qui s'étend au moins partiellement le long de la section axiale de la seconde gaine (301),
**caractérisé en ce que**
l'écrou-raccord (300) présente au niveau de la seconde gaine (301) des jours qui s'étendent au moins partiellement le long d'une section axiale de la seconde gaine (301) et, de manière contiguë à ceux-ci, des nervures sont formées dans la seconde gaine (301), sur lesquelles les sections du filet (305) sont agencées.

2. Système de raccordement (100) selon la revendication 1, **caractérisé en ce que**
la première gaine (201) est sensiblement en forme de cône dans la zone d'une extrémité du passage (202).

3. Système de raccordement (100) selon la revendication 1 ou 2, **caractérisé en ce que**
les sections du filet (305) forment au moins des flancs d'un filet (305) et en particulier les sections du filet (305) sont délimitées sur les zones des nervures.

4. Système de raccordement (100) selon la revendication 1, dans lequel la saillie (206) au niveau de la périphérie d'au moins une paire de liaison (104) est un épaulement (204), **caractérisé en ce que**
au moins une partie du contour extérieur de la section axiale (206) de l'épaulement (204) est agencée vers l'extérieur par rapport au passage (202) et vers l'intérieur par rapport à la zone de réception (302), est sensiblement un polygone inscrit dans un cercle et/ou un polygone circonscrit au cercle et le centre du cercle circonscrit du polygone inscrit dans un cercle et/ou le centre du cercle inscrit du polygone circonscrit au cercle sont agencés sensiblement au niveau du premier axe (A1) et/ou du second axe (A2).

5. Système de raccordement (100) selon la revendication 1 ou 4, dans lequel la saillie (206) au niveau de la périphérie d'au moins une paire de liaison (104) est un épaulement (204), **caractérisé en ce que**
au moins une partie du contour extérieur de la section axiale (206) de l'épaulement (204) est agencée vers l'extérieur par rapport au passage (202) et vers l'intérieur par rapport à la zone de réception (302), est sensiblement symétrique sous au moins un groupe de rotation discret par rapport au premier (A1) et/ou au second (A2) axe.

6. Système de raccordement (100) selon la revendication 1, 4 ou 5, **caractérisé en ce que**
la saillie (206) d'au moins une paire de liaison (104) est un ressort qui s'étend sensiblement le long d'un sens radial par rapport au premier (A1) et/ou second (A2) axe.

7. Système de raccordement (100) selon la revendication 1, ou 4 à 6, **caractérisé en ce que**
la saillie (206) d'au moins une paire de liaison (104) est un ressort qui s'étend sensiblement le long d'un sens azimutal par rapport au premier (A1) et/ou second (A2) axe.

8. Système de raccordement (100) selon l'une des revendications 1, ou 4 à 7, **caractérisé en ce que**
la saillie (206) d'une première paire de liaison (104) et la saillie (206) d'une deuxième paire de liaison (104) sont sensiblement identiques et leur agencement est sensiblement symétrique à une symétrie d'axe au niveau du premier (A1) et/ou du second (A2) axe.

9. Système de raccordement (100) selon l'une des revendications 1, ou 4 à 8, **caractérisé en ce que**
la saillie (206) d'une troisième paire de liaison (104), la saillie (206) d'une quatrième, la saillie (206) d'une cinquième paire de liaison (104) et la saillie (206) d'une sixième paire de liaison (104) sont sensiblement identiques et leur agencement est sensiblement symétrique en rotation avec un angle de 90°, 180°, 270° et 360° par rapport au premier (A1) et/second (A2) axe.

10. Système de raccordement (100) selon l'une des revendications précédentes, **caractérisé en ce que**
au moins des parties du système de raccordement (100) sont fabriquées en un matériau qui est sélectionné dans un groupe qui comporte une matière duroplastique et thermoplastique et en particulier du sulfure de polyphénylène, polypropylène, poly-1-butène, chlorure de polyvinyle, chlorure de polyvinylidène, polyméthyle-métaacrylate, polyméthacrylate de méthyle, polyacrylonitrile, polystyrène, polysulfone, polyacétal, alcool de polyvinyle, acétate de polyvinyle, éthylène-acétate de vinyle (EVA), des mélanges d'éthylène-acétate de vinyle et polyéthylène, copolymère d'éthylène-acétate de vinyle (EVAC) tel qu'Elvax ou Evatane, des ionomères, matière plastique fluorée, polyéthylène, polyamide, en particulier un polyamide partiellement aromatique, polycarbonate, polyester, oxyde de polyphénylène, polysulfone, acétal de polyvinyle, polyuréthane, et polyéther chloré, nitrate de cellulose, acétate de cellulose, éther de cellulose, résine de phénol, résine d'urée, résine de thiourée, résine de mélamine, résine d'alkyle, résine allylique, silicone, polyimide, polybenzimidazole, résine époxy, matière plastique de caséine, polyuréthane réticulé, résine de polyester insaturée, matériaux antimicrobiens ou antiseptiques tels qu'argent à haute porosité, argent fabriqué sans ion, composés d'argent et en particulier microargent, composés libérant des ions métalliques, matériaux sans bisphénol A tels que Tritan, Terlux, acrylonitrile-butadiène-styrène, des combinaisons de ceux-ci et similaires.
